# EUROPEAN PATENT APPLICATION

(11) **EP 2 022 846 A1**
(43) Date of publication of application: **11.02.2009**
(21) Application number: 06843245.9
(22) Date of filing: 26.12.2006
(51) Int. Cl.: C12N 5/06, A61K 39/00, A61K 39/395, A61L 27/00, A61P 1/16, C07K 16/18, C12Q 1/02, C12Q 1/68

(54) **METHOD FOR ISOLATION OF STEM CELL**

(30) Priority: 02.05.2006 JP 2006128106
(71) Applicant: Stelic Institute of Regenerative Medicine, Minato-ku Tokyo 1060044 (JP)
(72) Inventor: YONEYAMA, Hiroyuki, c/o STELIC INST. OF REGENERATIVE MEDICINE, Tokyo 106-0044 (JP)
(74) Representative: Grünecker, Kinkeldey, Stockmair & Schwanhäusser Anwaltssozietät
(86) International application number: PCT/JP2006/325861
(87) International publication number: WO 2007/129428

(57) **Abstract**

The present inventors discovered for the first time that labeling cell nuclei makes it possible to efficiently isolate stem cells. Namely, it was elucidated that stem cells with labeled nuclei remained labeled even after cell division, and showed self-renewing and long-living abilities characteristic of stem cells. Efficient isolation of stem cells is possible, for instance, by labeling the nuclear of each cell in a heterogeneous cellular group followed by selecting those cells that maintain a labeled state even after cell division. The present invention provides methods for enabling visualization of stem cells of animal tissues in a living state by labeling using the essential functions of the stem cells, and methods for simply and easily isolating the stem cells in a fresh state without using at all genetic manipulation or artificial markers.

## Description

### Technical Field

The present invention relates to methods of isolating stem cells, stem cells isolated by the methods, and uses of the stem cells.

### Background Art

Research in the field of regenerative medicine is quickly expanding with the dawn of the 21 st century. Focused especially is the possibility of treating intractable diseases using stem cell transplantation. Indeed, such treatments are being clinically applied in some diseases, including myocardial infarction. At present, ES cells (embryonic stem cells), bone marrow-derived stem cells (mesenchymal cells), and tissue stem cells (somatic stem cells) are considered as major stem cell sources for transplantation, but each of these cells has advantages and disadvantages, and no consensus exists regarding decisive sources and their quality control. Although application in actual clinical fields is preceding progress in basic research, it is not at all known as to how the transplanted stem cells actually function in the body. Stem cells are considered to be heterogeneous cell populations, and to provide safe and effective stem-cell-transplantation therapy, it is essential to elucidate *in vivo* interactions between stem cells, interactions between stem cells and other cells, factors controlling stem cell dynamics and functions, etc. (Non-Patent Document 1).

Surprisingly however, decisive markers for prospectively identifying stem cells do not yet exist. At present, cells identified by any one of the following methods are used as "stem cells" for research: methods utilizing a combination of markers from juvenile cell markers picked up by researchers, including c-kit; methods expressing a detection-substance such as GFP using a promoter of a chosen gene; or methods utilizing cells which are differentiated after culture. Since these conventional methods are basically artificial, cells now used are not necessarily real stem cells found inside the body. It should be realized that studies on the fate and *in vivo* dynamics of the transplanted stem cells and the risk of cancerous changes thereof are lacking even now when stem-cell-therapy is being applied in certain clinical fields as mentioned above. Thus identification of prospective markers and *in vivo* dynamics of stem cells should be done as soon as possible. These are the most important research subjects for achieving safe and effective clinical introduction of not only transplantation therapy but also regenerative therapy in a wider sense.

Above all, the understanding of *in vivo* dynamics of tissue stem cells has clinically important meaning. Bromodeoxyuridine (BrdU)-labeling methods are now often used at animal experiment levels to identify cells that are close to tissue stem cells. Because BrdU is a thymidine analogue and is incorporated into DNA during the S phase of the cell cycle, it serves as an index of proliferating cells. According to Non-Patent Document 2, BrdU administered to newborn mice is incorporated into actively proliferating cells, and part of these cells remain labeled until the mice grow into mature adult mice: these cells are defined as label-retaining cells (LRC), and are considered to be close to stem cells. However, since techniques of destructing nuclear membranes with hydrochloric acid and such are essential in identifying BrdU-incorporated cells with "anti-BrdU antibodies", the conventional methods can only be used for assessment using histological slices of animals.

Thus, it is a surprising fact that methods for prospectively isolating stem cells are completely absent even now when studies on stem cells are actively carried out.
[Non-Patent Document 1] Ruth Kirschstein & Lana R Skirboll "Stem Cells: Scientific Progress and Future Research Directions", National Institute of Health, 2001, pp1-106.
[Non-Patent Document 2] Taylor et al., Cell, vol. 102,2000,451-461.

### Disclosure of the Invention

### [Problems to be solved by the invention]

A problem to be solved by the present invention is to provide methods of prospectively isolating stem cells, stem cells isolated by the methods, and pharmaceutical uses of the stem cells in therapy, or as vaccines and such.

### [Means for solving the problems]

It has been proposed that "cancer stem cells" which alone have proliferating activity exist, and therefore cancer is "a disease of stem cells" (Clarke MF. Cancer Chemother Pharmacol 2005, 56, S64-68). Conventional cancer treatment thus cannot completely cure tumors even if a regression can be achieved, because it does not target cancer stem cells. A new approach being studied is the possibility of completely curing cancer by developing anticancer drugs targeting cancer stem cells. Thus, to develop novel anticancer drugs, it is clinically highly significant to identify molecular markers of cancer stem cells, which have not yet been elucidated at all, similar to markers of physiological stem cells.

The present inventors discovered, for the first time, that it is possible to efficiently isolate stem cells by labeling cell nuclei (for example, nuclear membrane and such). Namely, it was elucidated that stem cells with labeled cell nuclei retained the label even after cell division, and displayed characteristics of stem cells such as having self-renewing and long-living abilities. For instance, efficient isolation of stem cells is possible by labeling the cell nuclei of each cell of a heterogeneous cell population followed by selecting those cells that still retain the label after cell division.

Further, the present invention makes it possible to visualize animal tissue stem cells in a living state by labeling them through the use of an essential function thereof, and also provides methods of simply and easily isolating animal tissue stem cells in a fresh state (namely, without cell culturing) without using genetic manipulation or any artificial markers.

The present invention includes methods of selectively labeling and isolating stem cells in a fresh state by applying their own functional characteristics in a natural state, and includes also the stem cells themselves isolated by the present methods.

If functionally isolated stem cells are used, one can easily identify antigens, antibodies, genes, cell lines, markers, and such, which are specific to the cells using known experiment techniques. These procedures would be impossible without the present invention. The construction of a lineup of stem-cell therapies for each organ or each individual was also enabled for the first time by targeting the thus-identified factors directed to various physiological and pathological stem cells. Thus the present invention will have a great impact not only in the development of clinical applications, but also in medical history.

The present invention relates to methods of prospectively isolating stem cells, stem cells isolated by the methods, and medical uses of the stem cells such as for treatments or as vaccines, and more specifically, provides the following inventions:
[1] A method of isolating a stem cell from a cell population, comprising the steps of:
   (a) labeling the cell nuclei of cells in the cell population;
   (b) allowing the cells to divide; and
   (c) selecting a cell comprising a labeled cell nucleus as a stem cell.
[2] A method of isolating a morbid stem cell, comprising isolating a stem cell by the method of [1] from a cell population that has been prepared from a disease tissue.

[3] A method of isolating a somatic stem cell, comprising isolating a stem cell by the method of [1] from a cell population that has been prepared from a tissue of an organ.

[4] A method of isolating a cancer stem cell, comprising isolating a stem cell by the method of [1] from a cell population that has been prepared from a cancer tissue.

[5] The method of any one of [1] to [4], comprising isolating the stem cell in a living state.
[6] A method of testing whether a test cell is a stem cell, comprising:
   (a) labeling the cell nucleus of the test cell;
   (b) allowing the cell to divide; and
   (c) judging the test cell to be a stem cell, if the cell nuclei of cells after division are labeled.
[7] A method of selectively labeling a stem cell, comprising labeling the cell nucleus of a cell.
[8] The method of [7], wherein the label is retained after cell division.
[9] The method of [7], wherein the above label is retained for at least one month or more.
[10] A method of selectively visualizing a stem cell in a living state, comprising contacting the cell with an agent for labeling cell nuclei.
[11] A method of observing a stem cell in a living state, comprising visualizing the stem cell by the method of [10].
[12] A method of identifying a stem cell marker, comprising:
   (a) isolating a stem cell by the method of [1]; and
   (b) identifying a marker in the isolated stem cell.
[13] A method of identifying a cancer stem cell-specific marker, comprising:
   (a) isolating a cancer stem cell by the method of [4]; and
   (b) identifying a marker in the isolated stem cell.
[14] A method of identifying an antigen of a stem cell, comprising:
   (a) isolating a stem cell by the method of [1]; and
   (b) identifying an antigen of the isolated stem cell.
[15] A method of identifying a gene specifically expressed in a stem cell, comprising:
   (a) isolating a stem cell by the method of [1];
   (b) comparing gene expression states between the stem cell and a control cell; and
   (c) selecting a gene whose expression state varies in the stem cell as a gene specifically expressed in a stem cell.
[16] A method of identifying a target gene for treatment of a disease, comprising:
   (a) isolating a morbid stem cell by the method of [2];
   (b) comparing gene expression states between the morbid stem cell and a control cell; and
   (c) selecting a gene whose expression state varies in the morbid stem cell as the target gene.
[17] The method of any one of [1] to [16], comprising labeling the nuclear membrane or a nucleic acid in the cell nucleus (or nuclei).
[18] A method of treating a disease relating to an organ, comprising transplanting the stem cell isolated by the method of [1] into the organ.
[19] A method of treating a disease relating to an organ, comprising transplanting an organ that has been differentiated from the stem cell isolated by the method of [1].
[20] A method of antibody therapy, comprising administering an antibody against the stem cell isolated by the method of [1] to an individual.
[21] A method of gene-silencing therapy, comprising suppressing the expression of the gene identified by the method of [15] or [16].
[22] A method of screening for an anticancer agent, comprising:
   (a) isolating a cancer stem cell by the method of [4];
   (b) contacting the cancer stem cell with a test compound;
   (c) detecting a state of cell proliferation or cell death of the cancer stem cell; and
   (d) selecting a compound that suppresses cell proliferation or enhances cell death of the cancer stem cell as compared to a control.
[23] A stem cell isolated by the method of any one of [1] to [5].
[24] The stem cell of [23], comprising the following characteristics (a) and/or (b):
   (a) having the self replicating ability; and
   (b) being live for at least one month or more.
[25] A stem cell whose labeled cell nucleus is still labeled after cell division.
[26] The stem cell of any one of [23] to [25], wherein the stem cell is a living cell.
[27] A somatic stem cell isolated by the method of [1] from a cell population that has been prepared from an organ.
[28] A stem cell line isolated by the method of [1] from a cell population that has been established *in vitro*.
[29] The stem cell line of [28], wherein the stem cell line is a cancer cell.
[30] A stem-cell-specific cell line established from the stem cell that has been isolated by the method of [1].
[31] A cellular antigen of a stem cell, which is isolated by the method of any one of [1] to [5].
[32] An antibody that specifically recognizes a stem cell that has been isolated by the method of any one of [1] to [5].
[33] An artificial organ, which is constructed as a result of differentiation of a stem cell that has been isolated by the method of [1].
[34] A pharmaceutical composition, comprising as an active ingredient a stem cell isolated by the method of [1].
[35] A reagent for labeling stem cells, comprising as an active ingredient an agent for labeling cell nuclei.
[36] A stem-cell vaccine, comprising as an active ingredient an antigen of a stem cell isolated by the method of [1].
[37] An antibody pharmaceutical, comprising as an active ingredient an antibody against a stem cell isolated by the method of [1].
[38] An agent for testing a disease, comprising as an active ingredient a stem cell marker identified by the method of [12] or [13].
[39] A method of producing a labeled stem cell, comprising a step of isolating a stem cell by the method of any one of [1] to [5].
[40] The method of [39], wherein the above stem cell is a living cell.
[41] A method of producing a stem-cell-specific cell line, comprising:
   (a) isolating a stem cell by the method of [1]; and
   (b) making an established cell line of the above stem cell.
[42] A method of producing an artificial organ, comprising allowing the stem cell isolated by the method of [1] to differentiate.
[43] A method of producing a stem-cell vaccine comprising an antigen of a stem cell as an active ingredient, which method comprises:
   (a) isolating a stem cell by the method of [1]; and
   (b) identifying an antigen of the above stem cell.
[44] A method of producing an antibody against a stem cell, comprising:
   (a) isolating a stem cell by the method of any one of [1] to [5]; and
   (b) producing an antibody whose antigen is the stem cell, or a part of the stem cell.
[45] A method of producing an anticancer antibody against a specific organ, comprising:
   (a) isolating a cancer stem cell by the method of [1] from a cell population that has been prepared from a cancer tissue present in the organ; and
   (b) producing an antibody whose antigen is the above cancer stem cell, or a part of the stem cell.

### [Effects of the invention]

Use of the stem cells isolated in a fresh state through the methods according to the present invention enables, for the first time, the analysis of stem cells in a state closer to that in a living body and makes it possible to identify stem-cell specific markers. Further, the methods also enable visualization of the *in vivo* dynamics of stem cells in a living individual, and thus are applicable to the diagnosis of the fate of stem cells, which is a risk factor in clinical application.

Furthermore, the present techniques can easily be applied to cancer cell lines which are established *in vitro* after tumor portions are collected from cancer-bearing patients, and enables, for the first time, the identification of cancer stem cells which lack decisive markers at present. The present methods are the only methods that can identify true markers of various stem cells.

The present invention completely changes the aspects of conventional stem cell research, which could only conduct retrospective analyses using artificial markers that are not necessarily essential, and makes it possible to prospectively conduct research in a state closer to a true living body. Further, (i) the present methods are the only methods that enable the establishment of true stem-cell markers by identifying antigens and genes of isolated stem cells, and (ii) "cell therapy" using the isolated stem cells themselves is expected to be safer and bring about stronger effects than the "cell therapy" carried out now using "cells after culture". Both of the above are tools that cannot be achieved without using the stem cells obtained by the present methods (the experiments of antigens, genes, antibodies, cell lines, and cell therapy can be easily repeated by methods known to those skilled in the art, if the cells themselves exist), and they can provide highly expansive and numerous research themes and treatment cases.

Further, construction of functional artificial organs is possible by adding (culturing) the stem cells isolated by the methods according to the present invention.

Regarding the liver for instance, the construction of an artificial liver has so far been difficult. Up to now, it was considered to be enough if each liver cell could properly produce albumin and excrete toxic substances. However, it is thought that the most important thing is actually how well the functional construction as hepatic cords (the arrangement/alignment of hepatocytes) is maintained, and whether the whole liver can excrete bile: the disregard for this aspect is considered to be the cause of repeated failures so far. Hitherto, studies on how to construct three-dimensional matrixes have actively been carried out, but after all, clinical application has turned out to be impossible unless "functional construction of hepatic cords and bile excretion as a whole liver" can be achieved. Even in this sense, the hepatocytes isolated by the methods according to the present invention were found to be capable of forming a hepatic-cord construct on a culture dish for the first time in the world, and are thus usable for making artificial organs in tissue engineering.

### Brief Description of the Drawings

[Fig. 1] Photographs of hepatic stem cells immunostained using the BrdU-labeling method.
[Fig. 2] Photographs showing the migration of hepatic stem cells during tissue repair. Hepatic stem cells (brown color: BrdU) multiplied and moved as a mass toward a necrotic area for repair. Blue color indicates type IV collagen.
[Fig. 3] Photographs showing the tumorigenesis by hepatic stem cells.
[Fig. 4] Photographs showing the labeling of hepatic stem cells in a living mouse.
[Fig. 5] Photographs showing the labeling of colonic stem cells in a living mouse.
[Fig. 6] Photographs showing the labeling of hepatic stem cells in a living mouse.
[Fig. 7] Photographs showing the isolation of hepatic stem cells. SYTO GREEN^{negative} cells did not show colony formation, but SYTO GREEN^{high} cells showed colony formation.
[Fig. 8] Photographs showing hepatic cord-like constructs formed by isolated hepatic stem cells.
[Fig. 9] Photographs showing the labeling of cancer stem cells during the culturing process of a cancer cell line.

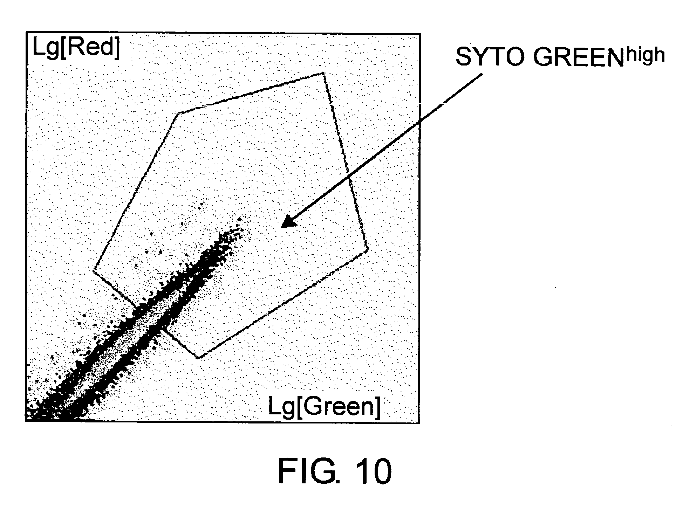

[Fig. 10] A figure showing the isolation of cancer stem cells from a cancer cell line.

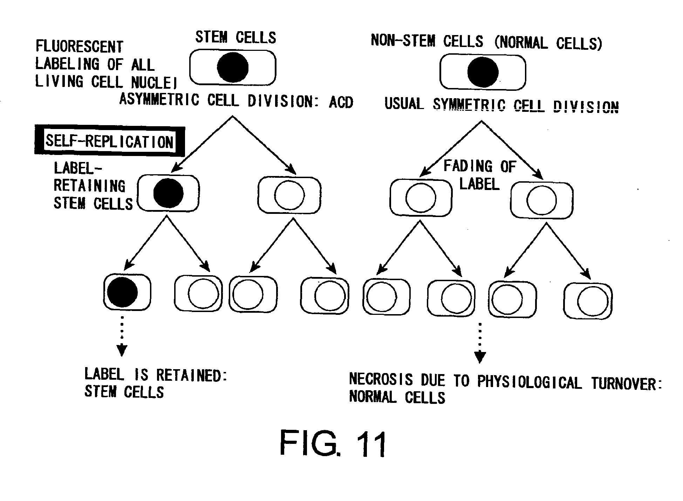

[Fig. 11] A schematic drawing showing the principle of the present invention.
[Fig. 12] Photographs showing the labeling of cancer stem cells in the liver in a living mouse.
[Fig. 13] Figures and photographs showing the isolation of cancer stem cells from a cancer cell line.

### Best Mode for Carrying Out the Invention

The present invention provides methods for isolating stem cells from cellular groups.

The term "stem cells" according to the present invention usually refers to stem cells in a living state (in some cases, such cells are referred to in the present description as "the stem cells according to the present invention"). The stem cells according to the present invention have the characteristic properties of being able to (i) self replicate, and (ii) live for a long period of time. These properties are maintained even after cell division. Since stem cells with labeled cell nuclei retain the label even after cell division, one can isolate stem cells by using the label as an index.

The stem cells which can be isolated by the methods according to the present invention include, for example, somatic stem cells and embryonic stem cells. More specifically, somatic cells include stem cells of endodermal, mesodermal, and ectodermal lineage. Endodermic tissues and organs include the thymus, thyroid, parathyroid, pharynx, bronchial tube, lungs, bladder, vagina, ureters, digestive organs (esophagus, stomach, small intestine, large intestine, liver, pancreas) and such. Mesodermal tissues and organs include bone marrow (= bone marrow stem cells or hematopoietic stem cells), adrenal cortex, lymph nodes and vessels, bone, muscle, heart, connective tissue of trunk (= mesodermal stem cells), blood vessels (= vascular endothelial stem cells), kidneys, and such. Ectodermal tissues and organs include the skin, central nervous system (= neural stem cells), adrenal medulla, pituitary gland, connective tissues of head and face, eyes, ears, and such. (Fuchs E & Segre JA. Cell 100:143-155, 2000)

A preferable mode of the methods according to the present invention for isolating stem cells include methods comprising labeling cell nuclei and selecting those cells that retain the label even after cell division.

A method according to the present invention is preferably a method of isolating a stem cell from a cell population, comprising the steps of:
(a) labeling the cell nuclei of cells in the cellular population;
(b) allowing the cells to divide; and
(c) selecting a cell comprising a labeled cell nucleus as a stem cell.

Although the above-mentioned step (a) is not especially restricted, the step is usually conducted by bringing cells into contact with a substance which can label cell nuclei (in some cases, described as "labeling agent").

The cell nuclei according to the present invention refer to, for example, the nuclear membrane or nucleic acids (comprising genomic DNA, histone, chromatin, and such), preferably the nuclear membrane. That is, the nuclear membrane of cells is labeled in a preferable mode of the present invention.

"Labeling" according to the present invention refers to labeling cell nuclei to an extent that the labeled cell nuclei are distinguishable from unlabeled normal cell nuclei. In other words, the "label" according to the present invention is not restricted in terms of labeling techniques and types of labeling substances, as long as it enables distinction from normal cell nuclei. In the present invention, the "labeling" includes, for example, labeling with dyes (pigments), labeling with fluorescent substances (pigments), labeling with enzymes, and labeling with radioactive substances, but labeling with dyes or fluorescent substances is preferred.

The substances usable for labeling cell nuclei (labeling agents) in the methods according to the present invention include, for example, commercially available fluorescent pigments. Specifically, SYTO GREEN-Fluorescent Nucleic Acid Stain (Molecular Probes) can be given as a suitable example. This substance is a low-affinity, nucleic acid-binding substance, and passively diffuses through membranes of living cells, resulting in the staining of nucleic acids (Chen A & McConnell SK, Cell 82: 631-341, 1995).

Other than the above-mentioned materials, commercially available fluorescent pigments, for example, Fluorescent Nanocrystals (QUANTUM DOT Inc.) and Cellstain-Hoechst 33258, Cyto-dye (Merk), and such are usable, but fluorescent pigments are not especially restricted thereto.

Magnetic beads that can bind to nucleic acids can also be given as a suitable example of the labeling agents according to the present invention.

The labeling agents according to the present invention are not especially limited as long as they can label cell nuclei (nuclear membrane, nucleic acids, and such), and arbitrary substances (pigments, drugs, reagents, and such) can be used.

In the methods according to the present invention, those skilled in the art can appropriately carry out the methods of labeling cell nuclei by using the above labeling agents, depending on the type of labeling agent. If a commercially available labeling agent is used, one can appropriately label cell nuclei according to the instructions attached. Labeling is usually carried out by adding an appropriate amount of labeling agent to a cell culture comprising a cell population to be used. If the above-mentioned SYTO GREEN is used as a labeling agent, cell nuclei can be labeled, specifically, by the method mentioned in the Example as described later.

Further, in the present invention, the substances capable of labeling cell nuclei (cell-nuclear-labeling agents) were found to be able to specifically label stem cells. In other words, a new use of cell-nuclear-labeling agents was discovered. Thus, the present invention provides reagents for labeling stem cells comprising a cell nuclear-labeling agent as an effective ingredient.

The "cell population" in the above-mentioned methods according to the present invention is not especially restricted in terms of tissues and organs from which they are derived, and includes, for example, cell populations prepared (derived) from normal tissues and organs, cell populations prepared from morbid tissues, and cell populations prepared from cancer tissues. They may also be a mixture of cell populations comprising plural species of cells prepared from various tissues, organs, and such. The "cell population" according to the present invention may in some cases be called "cell group" or simply "cells".

As cell populations used in the methods of the present invention, those prepared from various organs can usually be cited as a suitable example. So-called "somatic stem cells" can be isolated from the cell populations by the methods according to the present invention. The somatic stem cells according to the present invention are also called adult stem cells.

Somatic stem cells are undifferentiated cells generated within already-differentiated individual tissues, and can renew themselves as mentioned below. They can also generate, through differentiation, all individual cells within the tissue - the "parent body" from which the somatic cells were derived. Somatic stem cells in a living subject have the ability to replicate and generate cells identical to themselves as long as the subject is alive, and this characteristic is called "self-renewal". Somatic stem cells usually differentiate into precursor cells, a pioneer of the differentiation, and the precursor cells further differentiate to acquire particular shapes and grow into "matured" cells which have specific functions (for example, contraction of muscle cells, exchange of signals by nervous cells). Places where somatic stem cells exist are bone marrow, blood, cornea and retina of eyes, brain, skeletal muscle, dental pulp, liver, skin, luminal wall of digestive organs such as stomach and intestine, and pancreas. Information regarding somatic stem cells is largely obtained from researches on hematopoietic stem cells isolated from bone marrow and blood. Hematopoietic stem cells having the ability to generate blood have been widely studied, and have also been applied to treatment of various diseases. Until now, finding and isolation of such stem cells that can generate every cells of the body have not been achieved. Somatic stem cells are few in number, and it is very difficult to specify and separate/purify them. Further, it is difficult to secure a sufficient number of somatic stem cells needed for transplantation, and it is also impossible to make them infinitely replicate and grow by artificial cultivation.

Somatic stem cells have been found in all tissues that develop from the three primitive cell layers of embryonic germ cells (ectoderm, endoderm, mesoderm).

Somatic stem cells can proliferate in the body without undergoing differentiation for a long period of time (this characteristic is referred to as "long-term self-renewal ability (long-term self-replicating ability)"), and they can give rise to mature cells that have shapes and functions characteristic of a particular body tissue.

The organs from which stem cells can be isolated by the methods according to the present invention include all the organs existing in a living body, and specific examples include, brain, skin, hair follicle, eye, ear, tooth, nail, nose, tongue, fat, muscle, blood vessel, lymph vessel, nerve, lymph node, spleen, bone, cartilage, lung, heart, liver, pancreas, kidney, digestive tract, mammary gland, thyroid, parathyroid, adrenal gland, prostate, testis, ovary, uterus, bladder, and such.

By using the methods according to the present invention to isolate stem cells from these organs, somatic stem cells unique to each organ can be obtained.

If a cell population prepared from a disease tissue is used as the cell populations in the present invention, the methods according to the present invention enable the isolation of stem cells involved in diseases (morbid stem cells). The morbid stem cells according to the present invention refer to somatic stem cells that fall into a morbid state (for example, turning cancerous or becoming colonized by pathogens) by some cause and replicate for a long period of time while keeping themselves in the morbid state and simultaneously giving rise to morbid daughter cells.

By using the methods according to the present invention to isolate stem cells from tissues relating to various diseases, morbid stem cells which serve as targets for the treatment of each disease can be isolated. For instance, as mentioned later, it is possible to treat diseases by using siRNA and such which can suppress the expression of genes specifically expressed in morbid-stem cells (gene-silencing treatment).

Further, if a cell population prepared from cancer tissues is used as a cell population according to the present invention, the methods according to the present invention can isolate cancer stem cells. The cancer stem cells according to the present invention refer to somatic stem cells which become cancerous by some cause, and unlimitedly replicate for a long period of time as cancer cells while simultaneously giving rise to daughter cells (cancer cells) (Beachy PA et al. Nature 432:324-331, 2004; Clarke MF & Fuller M. Cell 124:1111-1115, 2006).

By using the methods according to the present invention to isolate stem cells from tissues relating to various cancers, cancer stem cells which can serve as targets of selective treatment for each cancer can be isolated. For instance, as mentioned later, it is possible to treat specific cancers by using siRNA and such which can suppress the expression of genes specifically expressed in cancer stem cells (gene-silencing treatment).

Further, without limitation on the species of individual organisms from which the cell populations derive, the methods according to the present invention can be carried out in any organism as long as it comprises stem cells. Although there is no limitation on the organism species from which the stem cells that can be isolated by the methods according to the present invention derive, humans, mice, hamsters, rats, dogs, monkeys, goats, pigs, and such, for instance, can be given as examples.

In the above-mentioned step (b), the cell division can usually be achieved by culturing cells. The "cell division" according to the present invention includes "cell replication" and "cell growth (proliferation)".

Those skilled in the art can appropriately culture desired cells according to the cell type.

The stem cells according to the present invention, which are isolated under conditions where viability is good, are preferably stem cells that proliferate by themselves and which are able to proliferate and differentiate regardless of the presence or absence of supplemental nutrients (see the Examples described later).

Although there is no particular limitation, the stem-cell culture according to the present invention can be carried out, for example, under usual culture conditions. Specifically it can be carried out under the conditions of culturing at 37°C for one week by exchanging a medium once every 16 hours or every two days under an atmosphere of 5% CO₂ in a medium of RPMI or Dulbecco's Modified Eagle's Medium/Ham's F12 Medium containing 10% fetal bovine serum, 100 U/mL penicillin G, 100 mg/mL streptomycin, and 4.5 g/L glucose.

Further, when actually clinically applying the isolated cells to cellular therapy, the following methods can be employed: for example (i) adding feeder cells (fibroblasts), (ii) adding growth factors such as insulin, leukemia inhibitory factor (LIF), epidermal growth factor (EGF), basic fibroblast growth factor (bFGF), hepatocyte growth factor (HGF), or platelet-derived growth factor (PDGF), or (iii) utilizing fibronectin or matrigel as a matrix for cell adhesion/spreading (three-dimensional culture).

In the above-mentioned step (c), the selection of cells comprising labeled cell nuclei is carried out by using the presence or absence of the label as an index. Those skilled in the art can appropriately select (sort) the objective cells (cells comprising labeled cell nuclei) by using the presence or absence of the label as an index, according to the type of substance used for labeling (labeling agent). For instance, when the used labeling agent is a fluorescent substance, the selection of stem cells can be conducted with a commercially available cell sorter by using the presence or absence of fluorescence as an index. More specifically, the stem cells according to the present invention can be selected by the methods described in the Examples mentioned later. The methods of selecting stem cells in the above-mentioned step (c) according to the present invention are not especially restricted to the above-mentioned method using a cell sorter.

When magnetic beads are used as a labeling agent according to the present invention, only the stem cells that have incorporated the magnetic beads can be simply and easily isolated by letting a cell suspension containing stem cells comprising the magnetic beads pass through a magnet column (Veritas Co., Miltenyi Biotec, and such). If nanomagnetic beads are used, the magnetic strength of stem cells and normal cells can be easily and simply distinguished as a result of cell division. Further, image tracking of stem cells in actual clinical settings becomes possible through MRI (magnetic beads MRI) by using nanomagnetic beads as the labeling agent according to the present invention.

In the present invention's isolation methods, cells for which the presence of a label is detected, that is, cells comprising labeled cell nuclei, are selected as stem cells.

The stem cells isolated by the methods according to the present invention have, preferably, the characteristic of being living cells.

Further, it is also possible to call the isolation methods according to the present invention as, for example, selection methods, separation methods, sorting methods, or purification methods of stem cells.

Herein below, methods of isolating mouse stem cells are shown as an example of the isolation methods according to the present invention, but the methods according to the present invention are not especially restricted thereto.

First, a fluorescent pigment for staining cell nuclei (nucleic acids, nuclear membrane, and such) is administered to mice, or added to cell strains. By this, the nuclei of all living cells are fluorescently labeled by one treatment. The fluorescent pigment becomes retained only in the nuclei of cells undergoing "self-renewal" (that is, stem cells) after one to two weeks, although some time lag occurs depending on the turnover rate of each organ and cell strain. The cells identified at this time further retain the label for at least one month, although the period varies depending on the intensity and toxicity of the fluorescent pigment. This method faithfully abides by the laws of nature, and is based on a mouse or cell-based perspective, rather than on a human perspective, wherein stem cells alone are accurately made luminous.

By this method, one can observe the anatomical location and dynamics of stem cells in each organ in a living individual.

Furthermore, one can quickly isolate just the fluorescence-labeled stem cells by using commercially available cell sorting apparatuses such as cell sorters (functional cell-sorting) from each cell suspension prepared by a known method, and culture them if necessary.

The stem cells isolated in the present invention include not only those under normal, that is, physiological conditions, but also those under morbid conditions such as cancer stem cells.

The present invention includes methods of producing labeled stem cells, comprising the step of isolating them by the methods according to the present invention.

Further, it is possible to test whether or not the desired cells are stem cells, by using as an index the point of whether or not cells with labeled cell nuclei retain the label even after cell division. In other words, the present invention provides methods of testing or determining whether or not a subject cell is a stem cell by using the presence or absence of labeled cell nuclei as an index.

A preferable embodiment of a testing method according to the present invention includes a method comprising the steps of:
(a) labeling the cell nucleus of a subject cell;
(b) allowing the cell to divide; and
(c) judging the subject cell to be a stem cell, if the cell nuclei of cells after division are labeled.

The above-mentioned "subject cell" refers to a cell to be subjected to the test (determination) of whether or not the cell is a stem cell. According to the above-mentioned methods, the subject cell is judged as a stem cell when the cell nuclei of post-division cells are labeled, while the subject cell is judged as a non stem cell when the cell nuclei are not labeled.

Further, stem cells are selectively labeled in the isolation methods according to the present invention. Thus, methods of selectively labeling stem cells are also included in the present invention. The methods are usually methods comprising a step for labeling cell nuclei of cells.

The stem cells labeled by the above-mentioned methods are still labeled after cell division, and the label has the characteristic of being retained for a long period of time. The "long period of time" refers to a period significantly longer than the case when normal cells are labeled, and refers to, for example, a period of two days to one week or more, preferably two weeks or more, more preferably three weeks or more, and further preferably, one month or more.

For instance, labeling substances (fluorescent pigments and such) are considered to disappear in normal cells after two repeats of cell division. Thus, although the period varies depending on the type of organ and cell, it is possible to discriminate the stem cells according to the present invention from normal cells by using the presence or absence of labeling substances as an index, within about three days in the case of intestinal tract, and within about five days in the case of skin. While at least one division is sufficient for the cell division in the above step (b) according to the present invention, two or more divisions are preferable.

The methods according to the present invention enable the labeling of stem cells in a living state. Namely, the present invention relates to methods of selectively visualizing stem cells in a living state. A preferable mode of the methods is a method comprising the step of contacting cells with a cell nuclei-labeling agent. Here, "visualization" according to the present invention is not necessarily restricted to observation by the naked eye. "Visualization" according to the present invention comprises cases such as observing or imaging by using various detection equipments.

Those skilled in the art can appropriately select means for observing cells labeled with staining substances, fluorescent substances, and such, according to the substances used for the labeling. If staining substances are used as a labeling agent, one can observe, for example, using a light microscope. Further, one can usually observe using a fluorescent microscope when fluorescent substances (pigments) are used for the labeling.

The present invention also relates to methods for observing the stem cells visualized by the methods according to the present invention in a living state. A preferable mode of the methods is a method comprising a step for visualizing stem cells by a method according to the present invention.

Diagnostic imaging becomes possible by visualizing labeled stem cells in a living individual and observing the dynamics thereof by a method according to the present invention.

The stem cells isolated by the methods according to the present invention are applicable to various uses. For instance, the identification of stem cell markers is possible by using the stem cells according to the present invention.

The present invention provides methods for identifying a stem-cell marker by using the stem cells isolated by the methods according to the present invention.

A preferable mode of the above-mentioned methods according to the present invention is a method for identifying stem cell markers comprising steps of:
(a) isolating a stem cell by a method according to the present invention; and
(b) identifying a marker in the isolated stem cell.

"Identifying markers" in the above-mentioned methods refers to finding a characteristic comprised by a stem cells isolated by a method according to the present invention, which characteristic enables discrimination from other cells. Specifically, the identification of cellular antigens, the production of cell-specific antibodies, and such can be given as examples.

For example, because labels (fluorescent pigments and such) are retained by the stem cells isolated by the present methods, they can be detected as bands by conducting a gel-shift assay followed by Western blotting with antibodies against the fluorescent pigments. Thus, it is considered to be possible to actually analyze the antigens by MAS analysis and such. If antigens are identified, polyclonal antibodies and monoclonal antibodies against them can be prepared by general, known techniques, and the antibodies themselves serve as markers.

Further, if sugar chains on cell surface are identified, they serve as very effective markers. Furthermore, it is possible to make monoclonal antibodies by immunizing animals of other species with the stem cells themselves which are isolated by the methods according to the present invention. In this case, it is possible to establish antibodies that can be used for immunostaining or have the function of eliminating cells.

An early diagnosis of various cancers, for example, becomes possible by utilizing the markers of cancer stem cells (cancer stem cell-specific markers) isolated by the methods according to the present invention. Namely, the cancer-stem-cell-specific markers according to the present invention are useful as cancer-diagnostic markers.

The present invention comprises also methods of identifying antigens of stem cells or methods of making antibodies against stem cells (preferably stem-cell-specific antibodies) by using the stem cells isolated by the methods according to the present invention.

A method of identifying the antigens of stem cells according to the present invention is, for example, a method comprising the steps of:
(a) isolating stem cells by the methods according to the present invention; and
(b) identifying antigens in the isolated stem cells.

Further, one can obtain cancer stem cell-specific markers, if one identifies the cellular markers of cancer stem cells isolated from cell populations prepared from cancer tissues by the methods according to the present invention.

The markers identified by the methods according to the present invention (for example, sugar chains and antibodies) are useful in actual clinical settings as a diagnostic marker. For instance, diagnostics for early detection of cancers are possible by utilizing the obtained cancer stem cell-specific sugar chain markers and/or antibodies and examining their amount in blood or urine samples. Further, diagnostic imaging for medical examinations is also possible by using general diagnostic imaging equipments such as CT, MRI, PET/SPECT for subjects to whom cancer stem cell-specific sugar chain markers or antibodies labeled based on nuclear medicine are administered. Furthermore, one can detect label-retaining stem cells by imaging using fluorescence-detecting imaging equipments (for example, a LED laser microscope and CCD camera) two to three weeks after the administration of labeling substances to subjects, when substances which are safe for administration into a living body (for example, safe fluorescent-nucleic-acid-labeling substances) are used as labeling agents according to the present invention. If an abnormal mass is detected at this time, the mass can be diagnosed as a very-early stage cancer. For example, if labeling is conducted prior to stomach/large intestine camera examinations, simple and easy detection, diagnosis and early treatment of a very-early stage cancer are possible.

Furthermore, the present invention relates to methods of identifying genes specifically expressed in stem cells (stem cell-specifically expressed genes) by using the stem cells isolated by the methods according to the present invention. For instance, treatment of diseases relating to stem cells is possible by suppressing the expression of genes obtained by the methods.

An object of suppressing gene expression includes, for example, (i) treating cancers or chronic infectious diseases (athlete's foot, hepatitis C, herpes zoster, and such) by knocking down/silencing the expression of genes expressed in cancer or morbid stem cells by means of siRNA/RNA interference and such, and (ii) identifying stem cell-specific biomarkers based on genes specific to stem cells, when such genes are found.

A preferable mode of the above-mentioned methods according to the present invention is a method of identifying a gene specifically expressed in a stem cell, comprising the steps of:
(a) isolating a stem cell by a method according to the present invention;
(b) comparing gene expression states between the stem cell and a control cell; and
(c) selecting a gene whose expression state varies in the stem cell as a stem cell-specifically expressed gene.

"Control cells" in the above-mentioned methods usually refer to cells other than stem cells, and include, for example, SYTO GREEN-negative cells and such.

In the above-mentioned step (c), genes whose expression state is augmented in stem cells are preferably selected.

Further, identification of target genes for treating diseases is possible by conducting the above-mentioned methods by using morbid stem cells that have been isolated by the methods according the present invention from cell populations prepared from diseased tissues.

One example of the above-mentioned methods according to the present invention is as follows: first, when SYTO GREEN is used as a labeling agent according to the present invention, the difference in gene expression is examined among at least three kinds of cellular groups consisting of a SYTO GREEN-positive group, a SYTO GREEN-negative group, and another cellular group serving as a so-called control (standard), which has been separately isolated. Then, those genes that are especially strongly or selectively expressed in the stem cell group that is strongly positive for SYTO GREEN are identified and are used as the target for treatment. As methods for gene expression analysis, general and known techniques such as micro-array (DNA chip) methods, ATAC-PCR methods, iAFLP methods, SAGE methods, and usual RT-PCR methods are usable.

The expression of the stem-cell-specifically-expressed genes, which are identified by the above-mentioned methods, can be suppressed by using, for example, RNA interference (RNAi), anti-sense molecules (nucleic acids), aptamer molecules (nucleic acids), and such.

If the sequence of genes whose expression is to be suppressed is known, it is possible to appropriately prepare nucleic acids that can bring about RNAi effects (siRNAs) to effectively suppress the expression of the genes. The RNA sequences constituting siRNAs can be appropriately selected, for example, by using commercially available software or such and usually based on the sequences of target genes. If the sequence of the RNA constituting the siRNA is determined, the siRNA or siRNA-expression vectors can be produced based on this sequence.

These siRNAs can be locally or systemically administered to a living body alone or in a form of drug prepared by mixing the siRNAs and atelocollagen, liposome, or such, to conduct gene-silencing therapy.

Thus, the present invention provides methods for gene-silencing therapy, comprising a step of suppressing the expression of the genes that have been identified by the methods according to the present invention. RNA interference (RNAi) methods, for example, can be utilized to suppress the expression of genes.

It is also possible to use techniques other than RNAi in the gene-silencing treatments according to the present invention, if they can suppress the expression of arbitrary genes. For instance, it is possible to suppress the expression of stem cell-specifically-expressed genes by using antisense nucleic acids, ribozymes, aptamers, and such.

Further, the present invention provides stem cells isolated by the methods according to the present invention. In other words, the stem cells themselves isolated by the methods according to the present invention are also included in the present invention.

The present invention includes methods for concentrating or purifying stem cells comprised in cell populations. Thus, the stem cells according to the present invention are not necessarily restricted to cell populations consisting of stem cells alone, and may be cell populations substantially comprised of stem cells. "Substantially comprised of stem cells" usually refers to a state where stem cells are comprised in a ratio higher than that of cell populations before carrying out the methods according to the present invention. Thus, cell populations substantially comprised of stem cells are also included in the stem cells according to the present invention. More specifically, such cell populations are, for example, populations in which the ratio of stem cells is usually 50% or more, preferably 80% or more, more preferably 90% or more, further preferably 95% or more, and most preferably 99% or more.

For instance, one can obtain cell populations comprising the stem cells according to the present invention at 80 to 90% purity by using usual cell sorting techniques. Further, one can obtain cell populations comprising the stem cells according to the present invention at a purity of 99% or more by setting a narrower-range gate (setting fractionation conditions on the monitor of a flow cytometer/cell sorter so as to secure strict isolation).

As preferable modes of the stem cells according to the present invention, for instance, the following stem cells or stem cell strains can be exemplified. Here, the stem cells according to the present invention usually have a characteristic of being live (viable cells).
(i) Morbid stem cells isolated by the methods according to the present invention from a cell population prepared from disease tissues.
(ii) Somatic stem cells isolated by the methods according to the present invention from a cell population prepared from organ tissues.
(iii) Cancer stem cells isolated by the methods according to the present invention from a cell population prepared from cancer tissues.
(iv) Stem cell lines isolated by the methods according to the present invention from a population of cell lines established *in vitro.*
(v) Stem cell lines (stem cell-specific cell lines) established or made from the stem cells isolated by the methods according to the present invention.

The establishment of cell strains/lines from the stem cells according to the present invention can be appropriately carried out, for instance, by general methods such as those described in "Robertson EJ. Biology of Reproduction 44:238-245, 1991".

As one example, one can establish cell lines from the stem cells according to the present invention by the following procedure:
(i) isolating stem cells; (ii) culturing the cells for three to five days with or without addition of feeder cells (fibroblasts); (iii) letting the cells form colonies, (iv) transferring the cells to a culture dish with a diameter of about 35 mm and culturing further for four to seven days; and (v) maintaining the cells through passaging.

Regarding cell culture media to be used, those skilled in the art can appropriately prepare an optimal medium by taking into account the cell type and such; and the medium of "100 mL DMEM + 4.5 g glucose + 0.5 mL NEAA + 1 mL nucleoside solution + 0.2 mL 2-mercaptoethanol solution" can be given as an example. Further, cytokines may be appropriately added to the medium, as needed.

Stem cell lines (for example, stem cell-specific cell lines) can be produced by establishing the stem cells according to the present invention by the above-mentioned methods. Such production methods are also included in the present invention. A preferable mode of the methods of producing a stem-cell line according to the present invention is a method comprising the steps of:
(a) isolating a stem cell by a method according to the present invention; and
(b) producing an established cell line of the stem cell.

Further, a preferable mode of the stem cells according to the present invention comprises the following characteristics:
(a) Self-renewing ability; and
(b) ability to live long

Furthermore, the stem cells according to the present invention preferably have the characteristic of retaining labels in their labeled nuclei, even after cell division.

The stem cells themselves according to the present invention can be used as pharmaceutical agents for treating diseases. Thus, the present invention provides pharmaceutical compositions comprising the stem cells isolated by a method according to the present invention as an active ingredient.

Because somatic stem cells are detectable in all organs, the pharmaceutical compositions according to the present invention target diseases of all organs (malfunctions and such).

The pharmaceutical compositions according to the present invention are expected to have therapeutic or preventive effects, for example, on neurodegenerative diseases such as Alzheimer's disease and Parkinson's disease, cardiovascular diseases such as myocardial infarction and myocardiopathy, renal failure, hepatic failure, diabetes, cancer, spinal cord injury, and autoimmune diseases such as multiple sclerosis. Further, an extremely wide range of applications other than the above-mentioned examples are possible; examples of such include application to intractable diseases like age-related macular degeneration, diabetic retinopathy, pulmonary adenomatosis, and inflammatory intestinal diseases, as well as applications to loss of eyesight, loss of hearing, loss of the sense of taste, alopecia, or reconstruction after surgery of breast cancer.

The present invention also includes cellular antigens of the stem cells isolated by the methods according to the present invention and antibodies that bind to the stem cells. The antigens are usable as stem cell vaccines. The antigens are preferably such that are specifically present in stem cells (stem cell-specific antigens). For instance, specific antigens obtained from cancer stem cells (for example, breast cancer) have an effect to prevent the emergence and growth of cancers (breast cancers and such). The specific antigens obtained from stem cells infected with herpesvirus have an effect to prevent the recurrence of herpes zoster in adulthood. Further, specific antigens obtained from stem cells of retina blood vessels have an effect to prevent the sudden blindness caused by diabetic retinopathy.

Additionally, the above-mentioned antibodies can be used as so called "antibody drugs". The antibodies are preferably those that specifically recognize stem cells. Stem cell-specific antibodies obtained from cancer stem cells (for example, breast cancer) have a cancer-treating effect (for example, breast cancer). Further, the stem cell-specific antibodies obtained from stem cells infected with hepatitis C virus have an effect in treating chronic type C hepatitis to which interferon therapy is ineffective and/or acute/fulminant hepatitis caused by the hepatitis C virus, and prevent chronic hepatitis from advancing to cirrhosis and hepatic cancer.

Thus, the present invention provides stem-cell vaccines comprising as an active ingredient an antigen of stem cells isolated by a method according to the present invention and antibody drugs comprising as an active ingredient an antibody against the stem cells according to the present invention (preferably an antibody specifically recognizing the stem cells according to the present invention).

Further, the present invention includes methods for producing stem-cell vaccines according to the present invention and antibodies against the stem cells according to the present invention.

A preferable mode of the methods of producing the stem cell vaccines according to the present invention is a method comprising the steps of:
(a) isolating a stem cell by a method according to the present invention; and
(b) identifying an antigen of the stem cell.

A preferable mode of the methods of producing antibodies according to the present invention is a method comprising the steps of:
(a) isolating a stem cell by a method according to the present invention; and
(b) producing an antibody whose antigen is the stem cell or a part thereof.

Further, it is possible to produce anti-cancer antibodies to specific organs by producing antibodies against cancer stem cells that have been isolated from cell populations prepared from cancer tissues by the methods according to the present invention.

It is usually possible to produce anti-stem-cell antibodies by general techniques for producing antibodies by using stem cells or parts thereof as antigens. For instance, one can prepare polyclonal antibodies by immunizing animals such as rabbits with purified stem cells according to the present invention or with peptides of a part thereof, collecting blood after a certain period of time, and removing blood clots. Further, one can prepare monoclonal antibodies by fusing antibody-producing cells of animals immunized with the above-mentioned cells or peptides with bone tumor cells, isolating resultant single-clone cells (hybridoma) producing the objective antibodies, and obtaining antibodies from the cells. The antibodies thus obtained are usable for the purification and/or detection of the cells according to the present invention. The present invention includes antibodies that bind to the stem cells according to the present invention. Utilization of the antibodies makes it possible to detect the location where the stem cells according to the present invention are present or to judge whether or not the stem cells according to the present invention are contained.

The form of the antibodies according to the present invention is not especially restricted, and, in addition to polyclonal antibodies and monoclonal antibodies, included are human antibodies, humanized antibodies made by gene recombination technique, low-molecular-weight antibodies, and, furthermore, their fragments and modified antibodies, as long as they bind to the stem cells according to the present invention or antigens thereof. In other words, the antibodies according to the present invention include polyclonal antibodies, monoclonal antibodies, chimeric antibodies, single-chain antibodies (scFv), humanized antibodies, and antibody fragments such as Fab, Fab', F(ab')2, Fc, and Fv. These antibodies may be modified with PEG and such as needed. Further, it is also possible to eliminate the need of secondary antibodies for detection by producing the antibodies as fusion proteins with β-galactosidase, maltose-binding protein, GST, green fluorescent protein (GFP), and such. The antibodies can be modified through the labeling with biotin or such so that they can be detected and/or collected with avidin or streptavidin.

Regarding the present invention's stem cells or peptides of parts thereof, which are to be used as antigens for sensitization to obtain antibodies, the animal species from which they are derived are not restricted. However, antibodies derived from mammals, for example mice and humans, are preferred; and especially antibodies derived from humans are preferred.

Antibodies against the stem cells according to the present invention suppress the function of the cells by binding to the stem cells according to the present invention, and are thereby expected to have therapeutic or improving effects on, for example, diseases that are caused by stem cell abnormalities. When using the obtained antibodies for the purpose of administering to human bodies (antibody therapy), human antibodies or humanized antibodies are preferred to reduce immunogenicity.

Further, the present invention relates to artificial organs that are made from the stem cells isolated by the methods according to the present invention. The artificial organs can be made by isolating stem cells usually existing in organ tissues by the methods according to the present invention, and artificially induce differentiation of the stem cells.

A desired organ can be produced from stem cells by appropriately using general techniques (for example, tissue engineering and such) according to the type of stem cells to be used.

For example, an artificial functional organ can be made by adding (culturing) the stem cells isolated by the methods according to the present invention to a model that is artificially constructed as a "pseudo organ" by using a matrix and such.

Methods for producing an artificial organ by the methods according to the present invention are also included in the present invention. A preferable mode of the methods for producing an artificial organ according to the present invention is methods comprising a step of allowing the stem cells according to the present invention to differentiate.

Further, the present invention relates to therapeutic or preventive methods comprising transplanting the stem cells according to the present invention or an artificial organ that has been differentiated from the stem cells into an individual.

For instance, the transplantation of the stem cells according to the present invention into an organ makes it possible to treat diseases relating to the organ. Further, the transplantation of an artificial organ according to the present invention makes it possible to prevent or treat diseases relating to the organ.

Further, it is possible to prevent or treat diseases by administering the stem-cell vaccines or antibodies according to the present invention to an individual.

Furthermore, the present invention enables the screening of anticancer agents or candidate compounds thereof by means of comparing states of cell growth and/or cell death using the cancer stem cell lines (cancer stem cell-specific cell lines) isolated by the methods according to the present invention and/or usual cancer cell lines. For examples, compounds that suppress the expression of genes specifically expressed in cancer stem cells are expected to serve as anticancer agents.

A preferable mode of the present invention's methods of screening for an anticancer agent is a method comprising the steps of:
(a) isolating a cancer stem cell by a method according to the present invention;
(b) contacting the cancer stem cell with a test compound;
(c) detecting a state of cell proliferation or cell death of the cancer stem cell; and
(d) selecting a compound that suppresses cell proliferation or enhances cell death of the cancer stem cell as compared to a control cell.

The test compounds used for the present methods are not especially restricted. They may be, for example, single compounds such as natural compounds, organic compounds, inorganic compounds, proteins, and peptides, and may be chemical compound libraries, expression products of gene libraries, cell extracts, cell culture supernatants, products of fermentation microorganisms, extracts of marine organisms, or extracts of plants; however, the compounds are not restricted thereto.

"Contact" of cells with a test compound is usually conducted by adding the test compound to a cell culture medium, but is not restricted to this method. When the test compound is a protein and such, the "contact" can be carried out by introducing a DNA vector that can express the protein in the cells.

The state of cell proliferation or cell death of the above-mentioned step (c) can be assessed, for example, by culturing the stem cells following step (b) and using as an index the number of living stem cells contained in the culture medium. The cell growth is judged as being suppressed, or the cell death is judged as being enhanced when the number of living cells decreases or the degree of cell growth declines as compared to a control.

Regarding the "control" in the screening methods of the present invention, those skilled in the art can appropriately choose (set) a suitable "control" according to the mode for carrying out the methods. For example, stem cells not contacted with test compounds or normal stem cells can be used as a "control". Namely, the "control" according to the present invention includes any one that makes it possible to judge the presence of the effects of test compounds.

As mentioned above, the present invention provides pharmaceutical agents (pharmaceutical compositions), comprising the present invention's stem cells, stem-cell vaccines, or antibodies against the stem cells.

The pharmaceutical agents according to the present invention can also be prepared as pharmaceutical formulations by known pharmaceutical manufacturing methods. For example, the pharmaceutical agents can be combined with suitable carriers or media generally used for pharmaceuticals, for example, sterile water, physiological saline, plant oils (examples: sesame oil and olive oil), coloring agents, emulsifiers (an example: cholesterol), suspending agents (an example: gum arabic), surfactants (an example: surfactants belonging to polyoxyethylene hydrogenated castor oil), solubilizers (an example: sodium phosphate), stabilizers (examples: sugars, sugar alcohols, and albumin), or preservatives (an example: paraben) to prepare medical formulations suitable for effective administration into a living body, such as injections, agents for intranasal absorption, agents for percutaneous absorption, and oral agents; among these, injections are preferred.

The administration to a living body can be conducted, for example, by intra-arterial, intravenous, or subcutaneous injection; it can also be transnasally, transbronchially, intramuscularly, or orally carried out by methods known to those skilled in the art. Although the dosage administered varies depending on the age, body weight, and symptoms of the patient as well as the administration method, one skilled in the art can suitably choose an appropriate dosage.

Further, the present invention provides kits for isolating and/or purifying stem cells, and kits for producing the stem-cell vaccines, antibodies against stem cells, or artificial organs according to the present invention.

The kits according the present invention can comprise as their constituents, for example, cell nuclei-labeling agents, culture media or solutions and such for culturing cells.

In a preferable mode of the kits for isolating and/or purifying stem cells according to the present invention, a kit comprises as its constituents at least a cell-nuclei-labeling agent and a culture medium or solution.

The kits according to the present invention can comprise the stem cells according to the present invention as a sample (a control). Furthermore, the kits according to the present invention can comprise reagents that are used in methods for detecting or selecting labeled stem cells.

The kits for producing the stem-cell vaccines and/or antibodies against stem cells according to the present invention preferably comprise as their constituents cell nuclei-labeling agents, media for culturing cells, and reagents for producing vaccines or antibodies.

As the above-mentioned "media", one can use media that are generally used for culturing cells. Those skilled in the art can easily know the basic compositions and such of the above-mentioned "media" from known literature or commercially available manuals and such.

Further, the kits according to the present invention can also comprise various reagents used in various methods such as cell sorting for isolating the cells according to the present invention. Additionally, specifications of the kits according the present invention, instructions for carrying out the methods, and such can be appropriately packaged.

An "individual" according to the present invention usually refers to humans, but can refer to animals other than humans. Namely, the animals are not especially restricted as long as they are usable for the isolation of stem cells, and usually are humans; however examples include non-human animals such as mice, hamsters, rats, dogs, monkeys, goats, and pigs. Stem cells isolated from *Drosophila*, nematode, and such are applicable in fields of basic research.

Furthermore, the present invention relates to methods for labeling stem cells, comprising administering cell nuclei-labeling agents to an individual. By these methods, labeling or detection of stem cells in a living body is possible.

Further, the above-mentioned methods according to the present invention can be used to distinguish stem cells from cells other than stem cells. It is usually possible to distinguish between stem cells and other cells by using as an index the labeling levels of cells that are labeled by the above-described methods according to the present invention. As shown in Examples described later, the above-described methods according to the present invention enable, appropriate distinguishing of, for example, three kinds of cells including cancer stem cells, normal stem cells, and normal cancer cells.

The methods according to the present invention also make it possible to selectively isolate stem cells from a cell population comprising plural kinds of cells.

Furthermore, one can screen for materials that selectively kill or injure stem cells or those that inhibit or enhance stem cell proliferation by using as an index the amount of stem cells labeled by a method according to the present invention.

All prior art references cited in the present specification are incorporated herein by reference.

### [Examples]

Herein below, the present invention will be specifically described using examples, but it is not to be construed as being limited thereto.

### [Example 1] An example of labeling tissue stem cells of living mice utilizing tissue sections: liver

Almost all somatic cells are actively proliferating in two-day old C57BL/6 mice (CLEA Japan, Inc.). In order to label the nuclei of such proliferating cells, bromodeoxyuridine (BrdU; Sigma-Aldrich, 5 mg/mL, 20 µL/mouse) was subcutaneously administered three times in total at twelve-hour intervals, and a follow-up examination was conducted over time until the age of eight weeks. BrdU is a thymidine analogue, and is incorporated into DNA during the S phase of the cell cycle (Taylor et al. Cell 102:451-461, 2000). At various time points, mice were sacrificed and their livers were collected. A part of the second lobe of the collected livers was embedded in a embedding media for freezing, the OCT compound (Miles), to make frozen blocks using liquid nitrogen. 6 µm-thick sections were made from the blocks using a cryostat (Microm). The obtained sections were fixed with acetone (Wako) for ten minutes, washed with a phosphate buffer solution, and immunostained by using a BrdU-staining kit (Zymed) according to the document attached thereto. The cells which incorporated BrdU were observed with a light microscope (Leica).

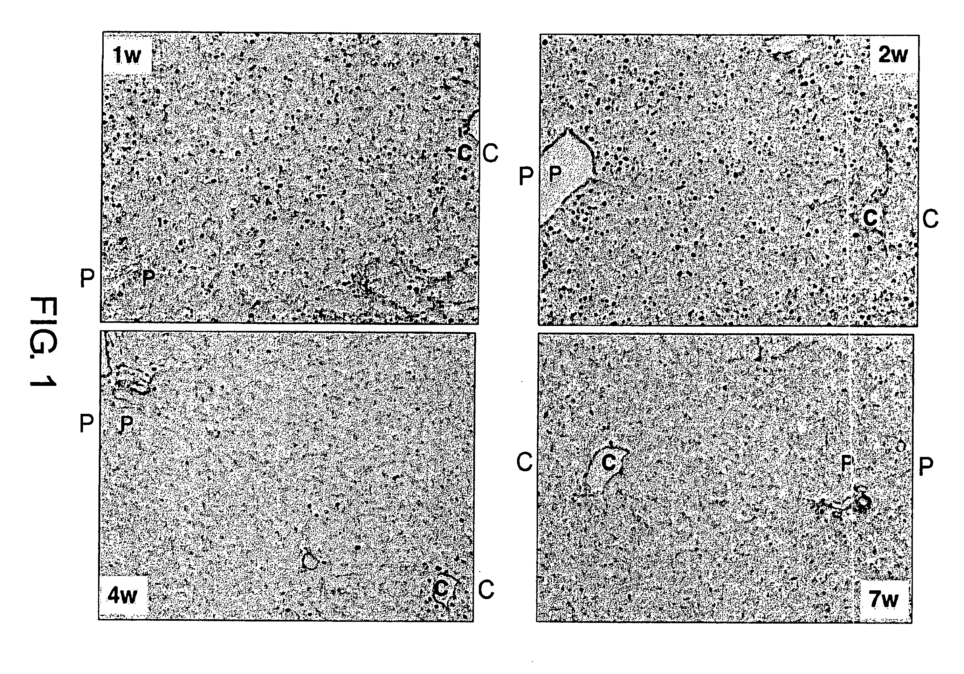

A typical example of the obtained immunostained images of liver tissue is shown in Fig. 1. While almost all liver cells retained incorporated BrdU in the liver at a week after birth, such cells drastically decreased at the age of two weeks and only a few were detected at four weeks. These BrdU-label-retaining cells were detected during an observation period of at least eight weeks, although the number was few. In sum, it was shown that almost all liver cells disappeared as a result of physiological turnover (metabolisms such as cell division and differentiation) and were replaced with new liver cells. On the other hand, because at least a few % of the cells of the whole liver existed as BrdU-label-retaining cells, these label-retaining cells turned out to be a small group of cells that fulfill the characteristic of stem cells remaining in organs for a long period of time.

### [Example 2] Evidence of tissue stem cells: liver repair

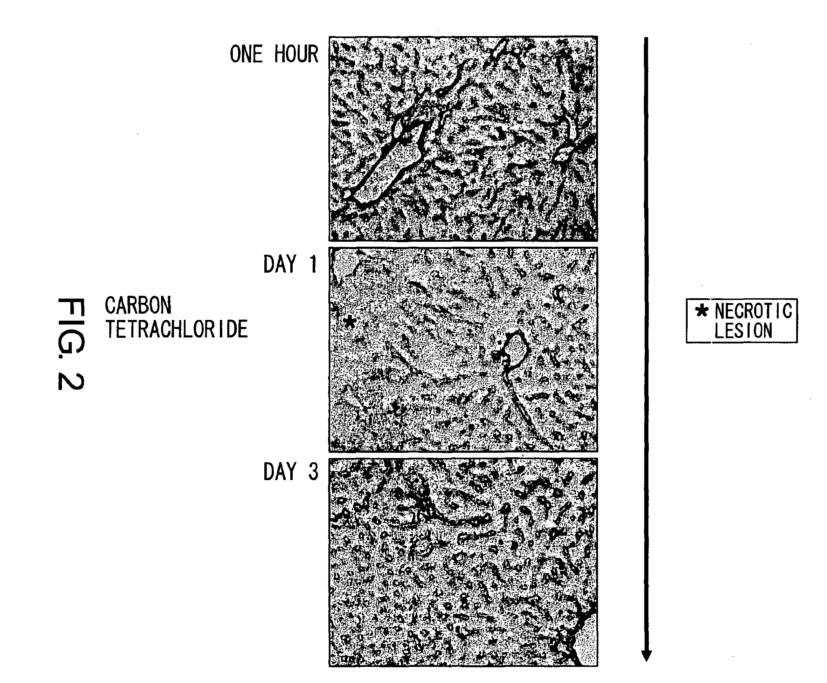

Carbon tetrachloride (50 µL/100 g body weight; Sigma-Aldrich) was intraperitoneally administered to the eight-week old mice obtained in Example 1; and immunostained images of the liver was examined over time by the method same as that in Example 1. This experimental system has been most generally used as a liver injury/regeneration model (Morrison GR. Arch. Biochem. Biophys. 111:448, 1965), in which a wide necrosis of liver cells is induced followed by enhanced liver-cell regeneration. One day after the administration of carbon tetrachloride, a necrosis of liver cells was observed over a wide area on one hand, and a quick growth of remaining liver cells was observed on the other (Fig. 2). At three days when the regeneration of liver cells after injury became enhanced, an image of tissue stem cells that obviously increased as a mass and were repairing necrotic tissue was obtained. In view of this, the BrdU-label-retaining cells were considered to be tissue stem cells, even functionally.

### [Example 3] Evidence of tissue stem cells: formation of liver cancer

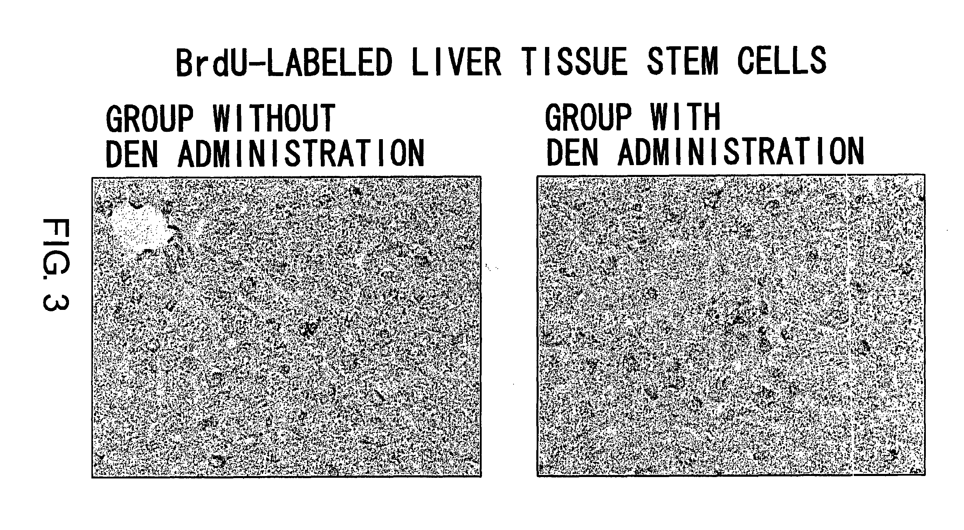

Hepatoma was induced in the four-week old male mice obtained in Example 1 by intraperitoneally injecting DEN (N-nitrosodiethylamine; 50 µg/mouse, Sigma-Aldrich) that is generally used in experiments as a chemical carcinogen (Yang X *et al*. Int J Cancer 118:1869-1876,2006). At three months after the administration of DEN, liver tissue sections were made by the same method as that of Example 1, and the dynamics of tissue stem cells were analyzed by the BrdU-staining method. Fig. 3 shows the results. Grossly visible tumor images were not yet formed, but super-early phase tumor-forming images centering at stem cells were histologically observed. The present Example showed that carcinogenesis starts from tissue stem cells.

### [Example 4] An example of fluorescent labeling of tissue stem cells in living mice: liver

Until now, the isolation and follow-up examination of the dynamics of stem cells in living individuals was impossible because only methods that injure nuclear membranes and nucleic acids, such as hydrochloric-acid treatment, could be chosen to detect BrdU-labeled cells. Namely, the detection was only possible in tissue sections prepared after sacrificing the individual or in cell suspensions prepared from tissues. As a result of dedicated research, the present inventors invented novel methods for isolating stem cells, which overcome such technical problems.

In order to stain all living cell nuclei of two-day old C57BL/6 mice (CLEA Japan), a SYTO GREEN-Fluorescent Nucleic Acid Stain (Molecular Probes, 50 nM, 20 µL/mouse), which is a fluorescent pigment penetrating to nucleic acids and nuclear membrane, was subcutaneously injected three times in total at twelve-hour intervals, and a follow-up examination was conducted for the same mice over time until the age of eight weeks. SYTO GREEN is a low-affinity, nucleic acid-binding material, and passively diffuses through membrane of living cells, resulting in the staining of nucleic acids (Chen A & McConnell SK, Cell 82: 631-341, 1995); however, it has never been used until now in living mice.

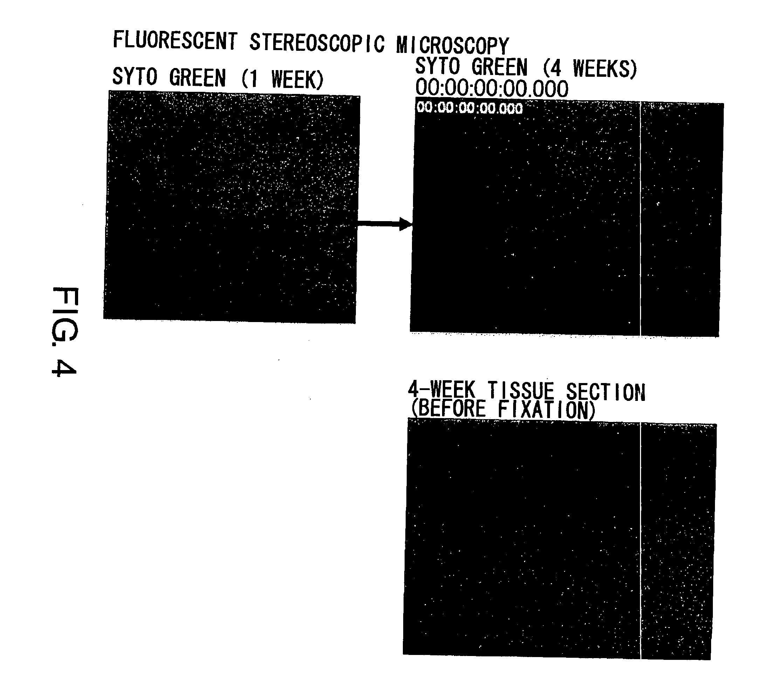

The anterior lobe of liver, which was exposed by median incision at the upper abdominal region after intraperitoneal anesthetization, was observed by using a fluorescent stereoscopic microscope (Leica). Fig. 4 shows a typical result at the age of one week. Almost all liver stem cell nuclei incorporated green fluorescent pigments. The present Example showed that nucleic acids and nuclear membrane of living cells could be stained even in a living individual by administering SYTO GREEN to a living body. The present labeling method can also be alternated with other Fluorescent Nanocrystals (QUANTUM DOT).

When the same mice were similarly observed at the age of four weeks, the SYTO GREEN-labeled liver cells drastically decreased to a few % of total liver cells, as in the typical example shown in Fig. 4. Although the SYTO GREEN-positive cells, that is, all liver cells, showed active proliferation/replication at two days after birth, almost all cells were demonstrated to be replaced with new liver cells through physiological turnover at four weeks. This result indicates that the SYTO GREEN-label-retaining liver cells at the age of four weeks are tissue stem cells having both the self-renewing ability and the ability to live long.

Tissue sections of liver were made by using the mice and a method similar to that of Example 1, and the cell distribution was confirmed by a fluorescent stereoscopic microscope. The SYTO GREEN label-retaining liver cells showed a distribution largely similar to that of BrdU-label-retaining cells of Example 1 even at the section level (Fig. 4).

### [Example 5] An example of fluorescent labeling of tissue stem cells in living mice: large intestine

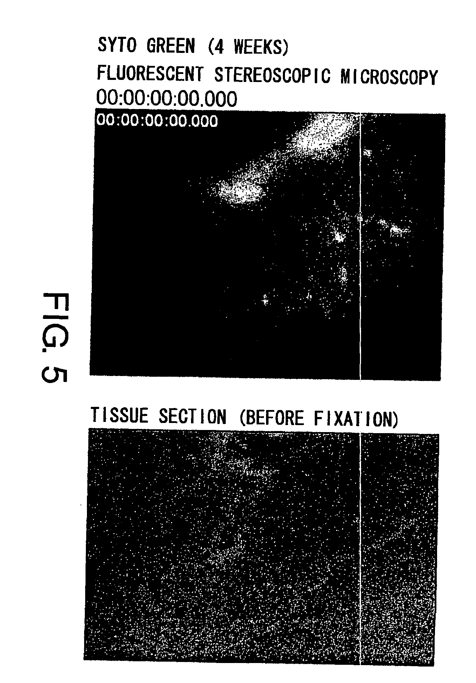

Other organs of the same mice at the age of four weeks of Example 4 were also examined. As an example, large intestine is shown because it has nearly been established that stem cells of the large intestine are located at the bottom crypt region (review; Fuchs E et al. Cell 116: 769-778, 2004). Cells strongly positive for SYTO GREEN were observed here and there in the lumen of large intestine during the observation in a living individual by a method similar to that of Example 4 using a fluorescent stereoscopic microscope (Fig. 5). Further, when tissue sections of the large intestine were made by a method similar to that of Example 1 and were observed with a fluorescent stereoscopic microscope, the cells were detected at the bottom region of crypt as already reported (Fig. 5). It is shown that the present labeling method makes it possible for the first time to detect tissue stem cells in an individual while it is alive.

### [Example 6a] An application example of fluorescent labeling of tissue stem cells in living mice: liver

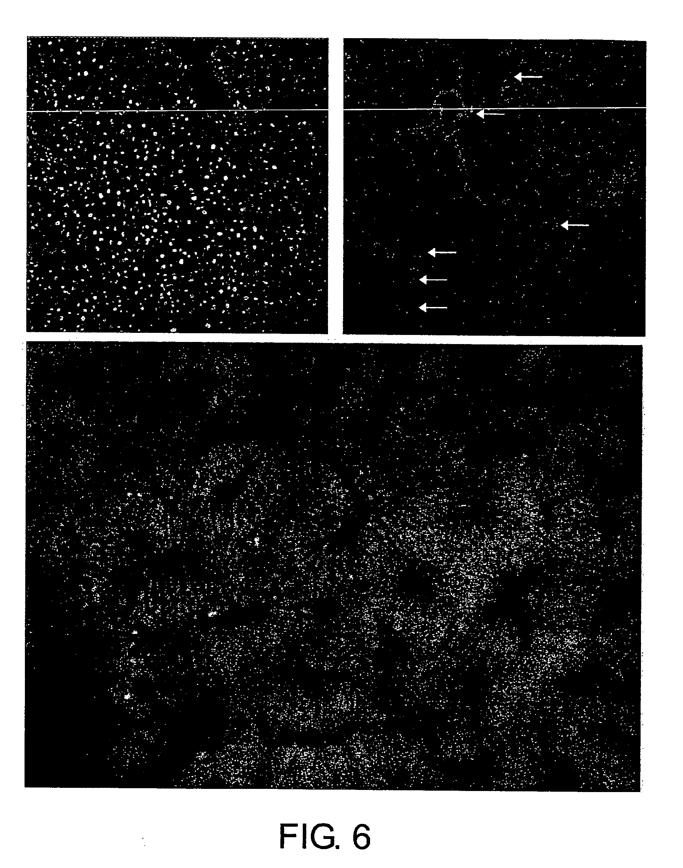

SYTO GREEN (50 nM, 100 µL/mouse) was intravenously injected once to two-week-old C57BL/6 mice (CLEA Japan), and four weeks later the mice were subjected to an observation with a fluorescent stereoscopic microscope in the living state by a method similar to that of Example 4. As shown in Fig. 6 (bottom), it was possible to label tissue stem cells of liver even in the present, simpler example. Further, tissue sections of liver were made by a method similar to that of Example 1, and immunofluorescent histological analysis was carried out by concurrently using type IV collagen. All cell nuclei were stained in the liver just after the labeling (Fig. 6 upper left panel), but tissue stem cells alone could be detected in the liver four weeks later (Fig. 6 upper right panel, arrows), when observed by using a confocal laser microscope. Namely, a detailed histological examination also confirmed the principle of the present invention.

### [Example 6b] An application example of fluorescent labeling of tissue stem cells in living mice: cancer stem cells of liver

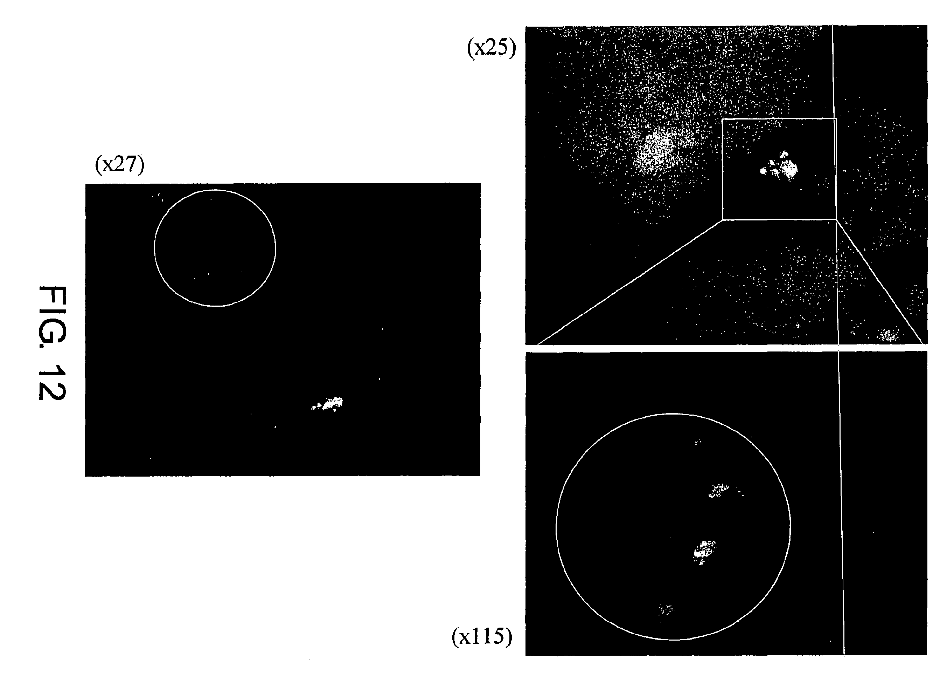

By a method similar to that of Example 3, DEN (50 µg/mouse) was intraperitoneally injected into four-week-old C57BL/6 mice to induce hepatoma. At nine months after the administration of DEN, SYTO GREEN (50 nM, 100 µL/mouse) was intravenously injected once to the mice, and two weeks later the mice were subjected to an observation with a fluorescent stereoscopic microscope in the living state by a method similar to that of Example 4. As shown in Fig. 12 (left panel), several cell-group masses highly bright in SYTO GREEN, which were thought to be small cancer stem cell clusters, were recognized in the small-size focuses that could be judged as early-cancer-forming focuses. On the other hand, quite a few such cancer stem cells were recognized in the cancer focus that has already become large enough to be obviously detectable by the naked eye (left panel, encircled with a broken line). Consistent with the principle of the present invention, it was made clear that retention of pigments was not observed in daughter cells that differentiated from cancer stem cells, that is, normal cancer cells. While isolated SYTO GREEN-positive cells were observed here and there, they were normal liver stem cells. Fig. 12 (right panel) shows an image under high magnification, which clearly shows that small clusters of SYTO GREEN-positive cancer stem cells and SYTO GREEN-negative daughter cells exist forming a mass in the cancer focus as shown within a broken line. This Example elucidated that the present invention is capable of easily discriminating three kinds of cells, cancer stem cells, normal stem cells, and normal cancer cells, according to the levels of brightness of these cells. That is, the present method enables clear detection of cancer stem cells by imaging at such an early phase when detection by the naked eye is not possible.

### [Example 7] Isolation of tissue stem cells

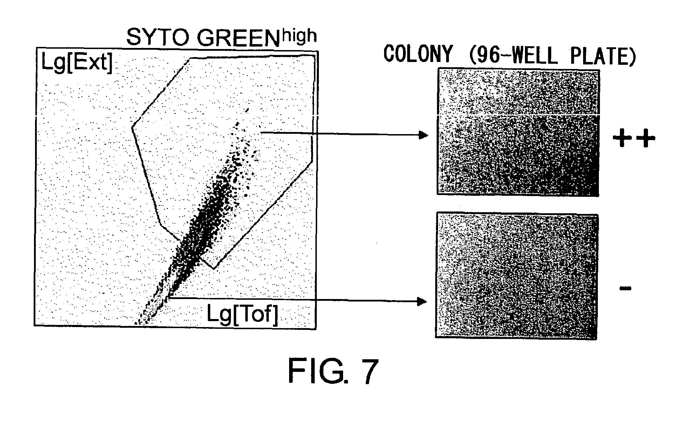

After collecting the livers of four-week old mice obtained by a method similar to that of Example 4, a liver cell suspension was prepared in an RPMI 1640 medium containing 10% fetal calf serum (herein below, described as FCS), 100 U/mL penicillin G, and 100 mg/mL streptomycin (all the reagents are from Gibco Inc.) as reported previously (Yoneyama et al. J. Exp. Med. 193:35-49, 2001). A freshly prepared liver cell suspension was immediately analyzed by using a flow cytometer (COPAS cell sorter, Union Biometrica Inc.). As shown in Fig. 7, the fluorescence of SYTO GREEN was still effective after separation of liver cells. Here, SYTO GREEN^{high} and SYTO GREEN^{negative} cells were sorted as single cells into each well of a 96-well plate. After culturing the cells for three days in a serum-free medium for liver cells (HepatoZYME-SFM, GIBCO Inc.) by using non-coated 96-well plates, colony formation was not detected for the SYTO GREEN^{negative} cells, but was detected for the SYTO GREEN^{high} cell group (Fig. 7). Thus, it became clear that SYTO GREEN^{high} cell population exerts an extremely high growth activity even without plate coating or addition of growth factors into the medium.

### [Example 8] Formation of hepatic-cord-like construct by isolated tissue stem cells

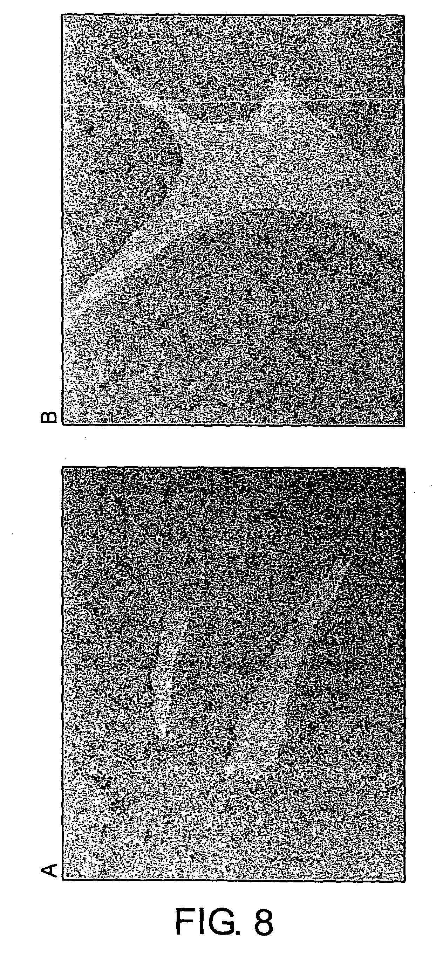

The SYTO GREEN^{high} cells which were isolated and propagated in Example 7 were seeded in a non-coated 10 cm culture dish, and were cultured for 24 hours. As a result, a monolayer of hepatic-cord-like construct very similar to that of liver tissue sections was quickly formed at the bottom of the dish (Fig. 8). The SYTO GREEN^{high} cells isolated by the present isolation method were shown to have the ability to construct a shape unique to the organ.

### [Example 9] An example of fluorescent labeling of cancer stem cells in human cancer cell lines: alveolar cell carcinoma

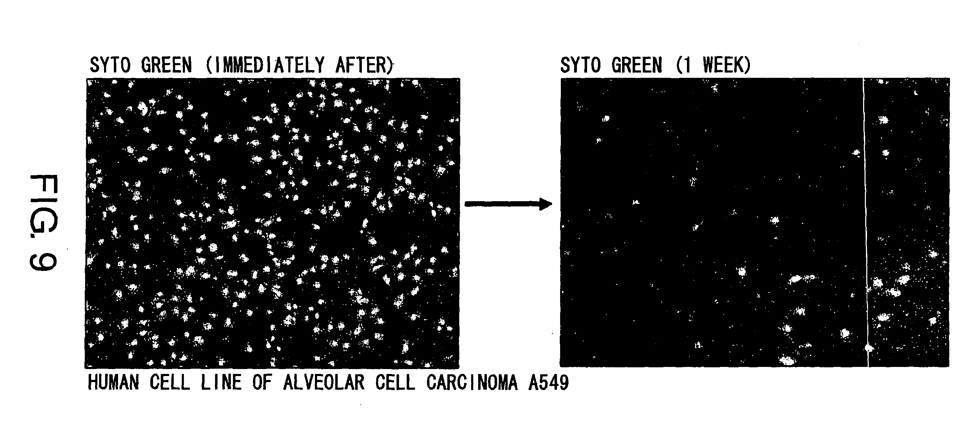

SYTO GREEN was added at a concentration of 5 ng/mL to a DMEM culture medium containing 10% FCS, 100 U/mL penicillin G, and 100 mg/mL streptomycin (all from Gibco Inc.) while culturing the cell line, A549 (J. Nat. Cancer Inst. 51:1417-1423, 1973), the alveolar cell carcinoma derived from human lung cancer. All cell nuclei were labeled one hour after the addition, whereas labeled cells decreased to several % one week after, even though the cells reached confluence (Fig. 9). This result shows that even in the process of cultured cell division and growth, cancer stem cells alone continue to self-replicate, consistent with the stem cell's definition, and SYTO GREEN bound to the nucleic acids are retained. Thus, it was elucidated that the present invention's principle is also applicable to normal cancer cell lines, making it possible to selectively and easily label cancer stem cells hidden in all cancer cell lines.

### [Example 10] Isolation of cancer stem cells from human cancer cell lines

The SYTO GREEN^{high} cells can be simply and easily isolated by a method similar to that of Example 7 by using a cell sorter.

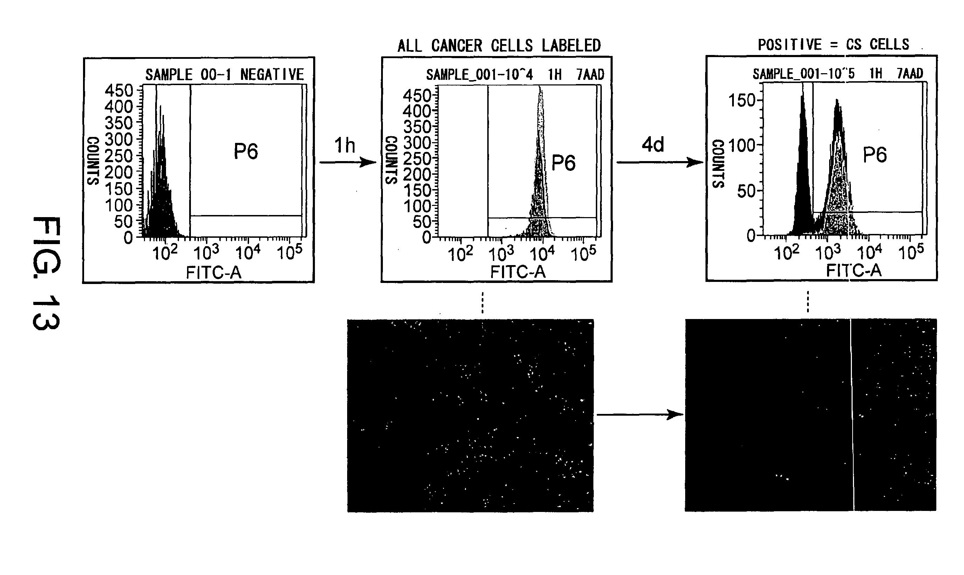

Furthermore, SYTO GREEN was added at a concentration of 5 ng/mL to a DMEM culture medium containing 10% FCS, 100 U/mL penicillin G, and 100 mg/mL streptomycin (all from Gibco Inc.) while culturing the cancer cell line, D54, derived from a human meningeal tumor, which is a brain tumor. All cell nuclei were labeled one hour after the addition, whereas labeled cells decreased to several % four days after, even though the cells reached confluence (Fig. 13). Since this progress can be followed clearly and quickly by observing a flow cytometry chart, one can simply and easily screen only substances that selectively injure and kill cancer stem cells when a test substance and such is added during the culture. Isolation by using a cell sorter is also easy.

### Industrial Applicability

The present invention expands at once the range of stem-cell research since the experimental techniques are extremely simple and easy, have a good reproducibility, and is practicable in any small laboratory in the world. Although at present there is no alternative but to use human cell lines, it will be possible to monitor stem cells in a living human individual when fluorescent pigments without toxicity to humans are developed. The present invention can realize super early diagnosis of cancer in the near future, by collaboratively working with the improvement and development of diagnostic imaging apparatuses (the hardware).

On the other hand, the present invention is realistically expected to contribute to: the development of antibody drugs and RNAi drugs, that selectively target for therapy "morbid stem cells"; and cancer therapy using a stem-cell vaccine for the first time in the medical history of humans. Further, development of safer and more effective cell therapy is expected by analyzing in detail the characteristics, culture conditions, *in vivo* fates , and such of isolated stem cells unique to each organ. Since liver stem cells easily form a sheet of hepatic cord by culturing, they are likely to be the best source that can be provided for constructing an artificial organ by tissue engineering.

## Claims

1. A method of isolating a stem cell from a cell population, comprising the steps of:
(a) labeling the cell nuclei of cells in the cell population;
(b) allowing the cells to divide; and
(c) selecting a cell comprising a labeled cell nucleus as a stem cell.

2. A method of isolating a morbid stem cell, comprising isolating a stem cell by the method of claim 1 from a cell population that has been prepared from a disease tissue.

3. A method of isolating a somatic stem cell, comprising isolating a stem cell by the method of claim 1 from a cell population that has been prepared from a tissue of an organ.

4. A method of isolating a cancer stem cell, comprising isolating a stem cell by the method of claim 1 from a cell population that has been prepared from a cancer tissue.

5. The method of any one of claims 1 to 4, comprising isolating the stem cell in a living state.

6. A method of testing whether a test cell is a stem cell, comprising:
(a) labeling the cell nucleus of the test cell;
(b) allowing the cell to divide; and
(c) judging the test cell to be a stem cell, if the cell nuclei of cells after division are labeled.

7. A method of selectively labeling a stem cell, comprising labeling the cell nucleus of a cell.

8. The method of claim 7, wherein the label is retained after cell division.

9. The method of claim 7, wherein the above label is retained for at least one month or more.

10. A method of selectively visualizing a stem cell in a living state, comprising contacting the cell with an agent for labeling cell nuclei.

11. A method of observing a stem cell in a living state, comprising visualizing the stem cell by the method of claim 10.

12. A method of identifying a stem cell marker, comprising:
(a) isolating a stem cell by the method of claim 1; and
(b) identifying a marker in the isolated stem cell.

13. A method of identifying a cancer stem cell-specific marker, comprising:
(a) isolating a cancer stem cell by the method of claim 4; and
(b) identifying a marker in the isolated stem cell.

14. A method of identifying an antigen of a stem cell, comprising:
(a) isolating a stem cell by the method of claim 1; and
(b) identifying an antigen of the isolated stem cell.

15. A method of identifying a gene specifically expressed in a stem cell, comprising:
(a) isolating a stem cell by the method of claim 1;
(b) comparing gene expression states between the stem cell and a control cell; and
(c) selecting a gene whose expression state varies in the stem cell as a gene specifically expressed in a stem cell.

16. A method of identifying a target gene for treatment of a disease, comprising:
(a) isolating a morbid stem cell by the method of claim 2;
(b) comparing gene expression states between the morbid stem cell and a control cell; and
(c) selecting a gene whose expression state varies in the morbid stem cell as the target gene.

17. The method of any one of claims 1 to 16, comprising labeling the nuclear membrane or a nucleic acid in the cell nucleus (or nuclei).

18. A method of treating a disease relating to an organ, comprising transplanting the stem cell isolated by the method of claim 1 into the organ.

19. A method of treating a disease relating to an organ, comprising transplanting an organ that has been differentiated from the stem cell isolated by the method of claim 1.

20. A method of antibody therapy, comprising administering an antibody against the stem cell isolated by the method of claim 1 to an individual.

21. A method of gene-silencing therapy, comprising suppressing the expression of the gene identified by the method of claim 15 or 16.

22. A method of screening for an anticancer agent, comprising:
(a) isolating a cancer stem cell by the method of claim 4;
(b) contacting the cancer stem cell with a test compound;
(c) detecting a state of cell proliferation or cell death of the cancer stem cell; and
(d) selecting a compound that suppresses cell proliferation or enhances cell death of the cancer stem cell as compared to a control.

23. A stem cell isolated by the method of any one of claims 1 to 5.

24. The stem cell of claim 23, comprising the following characteristics (a) and/or (b):
(a) having the self replicating ability; and
(b) being live for at least one month or more.

25. A stem cell whose labeled cell nucleus is still labeled after cell division.

26. The stem cell of any one of claims 23 to 25, wherein the stem cell is a living cell.

27. A somatic stem cell isolated by the method of claim 1 from a cell population that has been prepared from an organ.

28. A stem cell line isolated by the method of claim 1 from a cell population that has been established *in vitro.*

29. The stem cell line of claim 28, wherein the stem cell line is a cancer cell.

30. A stem-cell-specific cell line established from the stem cell that has been isolated by the method of claim 1.

31. A cellular antigen of a stem cell, which is isolated by the method of any one of claims 1 to 5.

32. An antibody that specifically recognizes a stem cell that has been isolated by the method of any one of claims 1 to 5.

33. An artificial organ, which is constructed as a result of differentiation of a stem cell that has been isolated by the method of claim 1.

34. A pharmaceutical composition, comprising as an active ingredient a stem cell isolated by the method of claim 1.

35. A reagent for labeling stem cells, comprising as an active ingredient an agent for labeling cell nuclei.

36. A stem-cell vaccine, comprising as an active ingredient an antigen of a stem cell isolated by the method of claim 1.

37. An antibody pharmaceutical, comprising as an active ingredient an antibody against a stem cell isolated by the method of claim 1.

38. An agent for testing a disease, comprising as an active ingredient a stem cell marker identified by the method of claim 12 or 13.

39. A method of producing a labeled stem cell, comprising a step of isolating a stem cell by the method of any one of claims 1 to 5.

40. The method of claim 39, wherein the above stem cell is a living cell.

41. A method of producing a stem-cell-specific cell line, comprising:
(a) isolating a stem cell by the method of claim 1; and
(b) making an established cell line of the above stem cell.

42. A method of producing an artificial organ, comprising allowing the stem cell isolated by the method of claim 1 to differentiate.

43. A method of producing a stem-cell vaccine comprising an antigen of a stem cell as an active ingredient, which method comprises:
(a) isolating a stem cell by the method of claim 1; and
(b) identifying an antigen of the above stem cell.

44. A method of producing an antibody against a stem cell, comprising:
(a) isolating a stem cell by the method of any one of claims 1 to 5; and
(b) producing an antibody whose antigen is the stem cell, or a part of the stem cell.

45. A method of producing an anticancer antibody against a specific organ, comprising:
(a) isolating a cancer stem cell by the method of claim 1 from a cell population that has been prepared from a cancer tissue present in the organ; and
(b) producing an antibody whose antigen is the above cancer stem cell, or a part of the stem cell.
